(19) Europäisches Patentamt
European Patent Office
Office européen des brevets

(11) Publication number: **0 092 787**
**B1**

(12) # EUROPEAN PATENT SPECIFICATION

(45) Date of publication of patent specification: **27.08.86**

(51) Int. Cl.$^4$: **C 07 C 103/29,** C 07 C 87/28

(21) Application number: **83103840.1**

(22) Date of filing: **20.04.83**

(54) **Process for the preparation of a phenylalkylaminoethylsalicylamide.**

(30) Priority: **26.04.82 US 371622**

(43) Date of publication of application:
**02.11.83 Bulletin 83/44**

(45) Publication of the grant of the patent:
**27.08.86 Bulletin 86/35**

(84) Designated Contracting States:
**AT BE CH DE FR GB IT LI NL SE**

(56) References cited:
**EP-A-0 009 702**
**DE-A-2 616 403**

**JOURNAL OF MEDICINAL CHEMISTRY, vol. 25, no. 6, June 1982, pages 670-679, J.E. CLIFTON et al.: "Arylethanolamines derived from salicylamide with alpha- and beta-adrenoceptor blocking activities. Preparation of labetalol, its enantiomers, and related salicylamides"**

(73) Proprietor: **SCHERING CORPORATION**
**2000 Galloping Hill Road**
**Kenilworth, New Jersey 07033 (US)**

(72) Inventor: **Gold, Elijah H.**
**10 Roosevelt Avenue**
**West Orange New Jersey 07052 (US)**
Inventor: **Babad, Esther**
**30 Conforti Avenue**
**West Orange New Jersey 07052 (US)**
Inventor: **Peer, Lydia**
**54 Warren Road**
**West Orange New Jersey 07052 (US)**
Inventor: **Chang, Wei K.**
**63 West Cedar Street**
**Livingston New Jersey 07039 (US)**

(74) Representative: **von Kreisler, Alek et al**
**Patentanwälte Von Kreisler-Schönwald-Fues-Keller Selting-Werner Deichmannhaus am Hauptbahnhof**
**D-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 092 787 B1**

Courier Press, Leamington Spa, England.

## Description

The substance 5-[1-hydroxy-2-(1-methyl-3-phenylpropyl)aminoethye]salicylamide, known as labetalol, is a well known antihypertensive agent as disclosed in U.S. Patent 4,012,444. Labetalol is a mixture of all four possible optically active stereoisomers which can be designated *R,R; R,S; S,R;* and *S,S,* according to the Cahn-Ingold-Prelog system. (−)-5-{(*R*)-1-Hydroxy-2-[(*R*)-1-methyl-3-phenylpropyl]aminoethyl}salicylamide, hereafter for convenience referred to as the *R,R* stereoisomer, is of particular interest. It possesses unique properties compared with the other isomeric forms, with the *(R,R)—(S,S)* and *(R,S)—(S,R)* diastereoisomers and with labetalol, enabling it to be used in the treatment of hypertension as discussed in detail in European Patent Application No. 79103473.9 (Publication No. 9702) incorporated by reference herein. The *R,R* stereoisomer substantially free of the other stereoisomers can be prepared by a series of process steps requiring chormatographic operations on diastereomeric intermediates in order to ensure that the desired sterically pure final product is obtained.

Thus European Patent Application No. 79103473.9 describes the stereoselective synthesis of the *R,R* stereoisomer and of its salts (in particular the hydrochloride) by a procedure including the step of chromatographically separating the desired isomer from a diastereomeric mixture.

We have now found that by employing certain stereospecific intermediates the *R,R* stereoisomer may conveniently be prepared by a process involving resolution of intermediates by salt formation instead of by chromatography.

The present invention therefore provides a method for the production of the *R,R* stereoisomer which does not require chromatography and which, in its preferred form, results in high yields. The process of this invention is a novel multistep process in which certain individual steps are themselves novel and in which certain of the intermediates are also novel.

We have found that, in the synthesis of the *R,R* stereoisomer, reduction of particular precursors, namely 2-(HO- or O-protected)-5-{N-[(*R*)-protected]-N-[(*R*)-1-methyl-3-phenylpropyl]glycyl}benzamides, affords more of the stereoisomeric intermediate with the desired *R* configuration at the newly formed hydroxymethyl chiral center than heretofore possible. Subsequent removal of the protecting groups yields a mixture consisting mainly of the desired *R,R* stereoisomer and a very minor amount of its *S,R* diastereoisomer. This mixture is readily resolved by salt formation to afford the desired compound. There is not need in the process of this invention for chromatographic separation of any intermediates.

The N-(*R*)-protecting group has the formula

|
Ar—CH—Y
*
(*R*)

wherein Ar is an aryl group, especially one having a single benzene ring, in particular a phenyl group ($C_6H_5$), and Y is a lower alkyl group having 1 to 6, especially 1 to 4, carbon atoms, e.g., an ethyl or in particular a methyl group. The preferred protecting group is the (*R*)-α-methylbenzyl group.

These N-*(R)*-protecting groups can be removed by hydrogenolysis, e.g. by means of hydrogen and a mild catalyst such as palladium on carbon, The O-protecting group is preferably one that can be removed simultaneously by hydrogenolysis, e.g., benzhydryl, benzyloxycarbonyl or in particular benzyl.

The 2-(O-protected)-5-{N-[(*R*)-protected]-N-(*R*)-1-methyl-3-phenylpropyl]glycyl}benzamides themselves can be formed from two intermediates, one of which is an optically active compound. This optically active compound is readily obtainable from a diastereoisomeric mixture (in which it is the major product) by non-chromatographic resolution.

Thus, the present invention provides a novel and unobvious method for the preparation of (−)-5-{(*R*)-1-hydroxy-2-[(*R*)-1-methyl-3-phenyl-propyl]aminoethyl}salicylamide.

In a preferred embodiment described below, the *R,R* stereoisomer of labetalol is produced by following the reaction sequence shown:

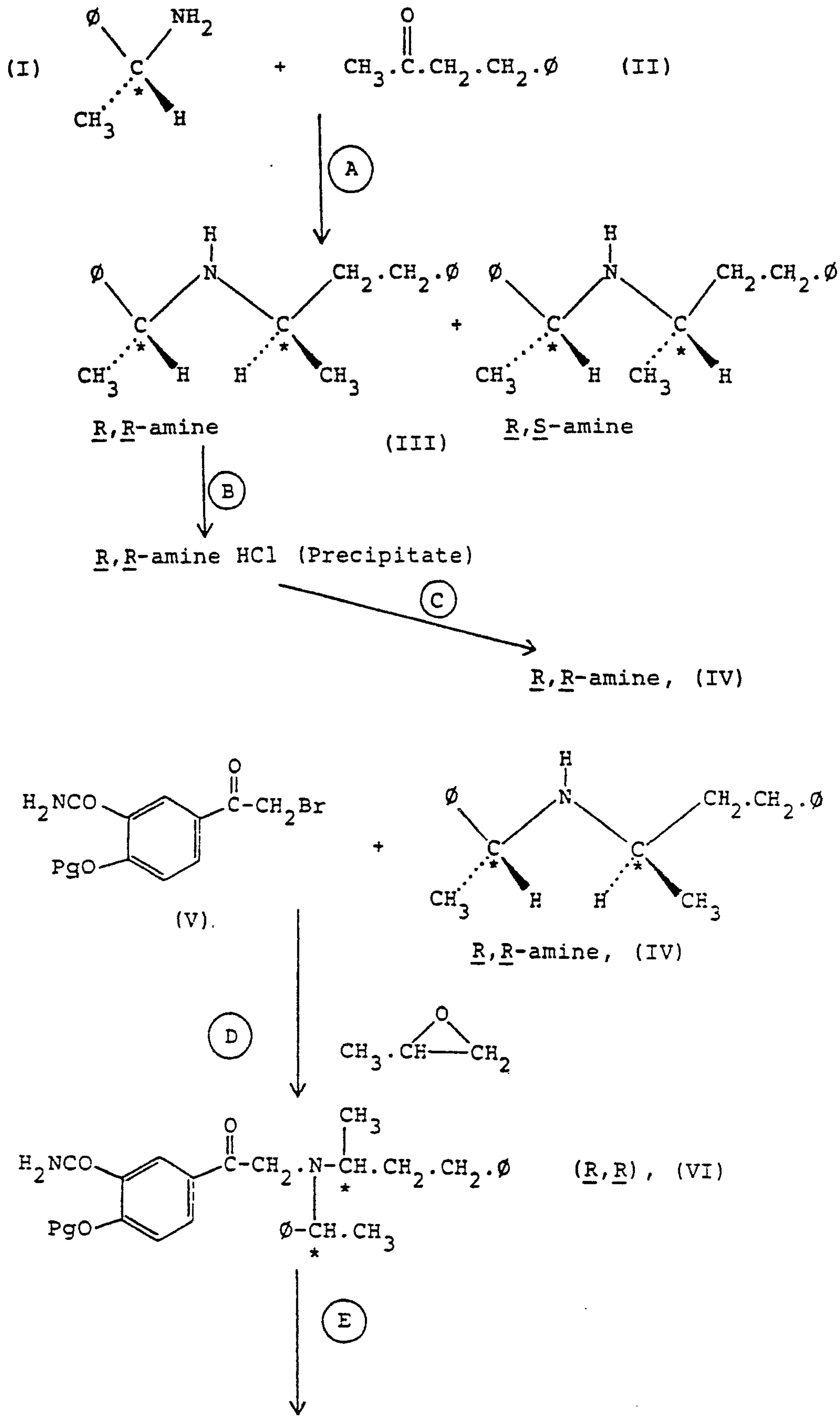


(I) (II)

R,R-amine (III) R,S-amine

R,R-amine HCl (Precipitate)

R,R-amine, (IV)

(V). R,R-amine, (IV)

(R,R), (VI)

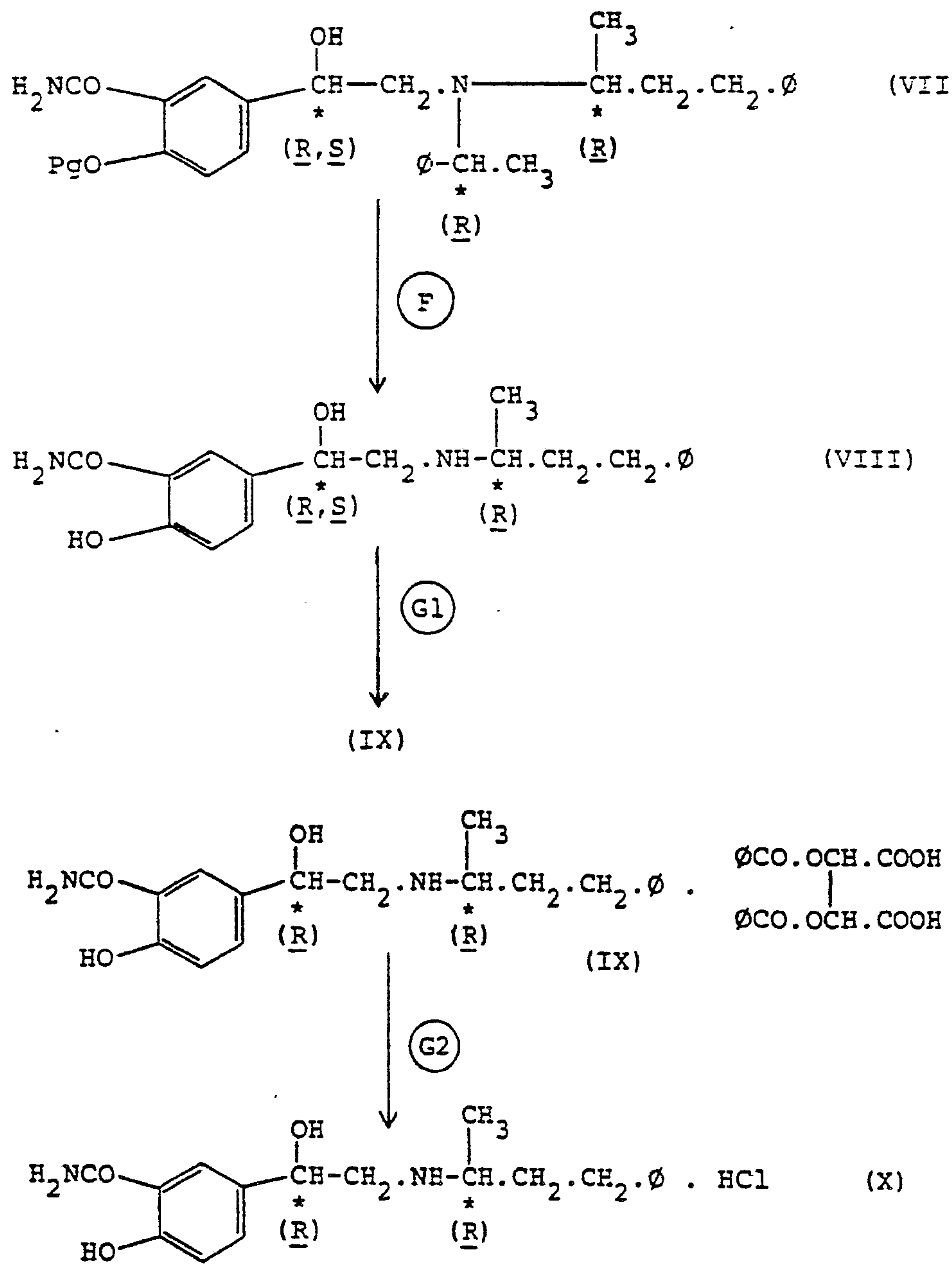


(VII)

(VIII)

(IX)

(IX)

(X)

In this reaction scheme:

"φ" shows an unsubstituted phenyl group ($C_6H_5$);

Asterisks indicate asymmetrically substituted carbon atoms;

"Pg" indicates a hydroxy protecting group, e.g. benzhydryl, benzyloxycarbonyl or especially benzyl;

compounds (IV), (VI) and (VII) are novel and are features of the present invention; and the steps A to G2 can be described in general terms as follows:

A: Condensation and reduction to a diastereoisomeric mixture of (*R,R*)- and (*R,S*)-amines, if desired in two successive stages but preferably in a single step of reductive condensation;

B: Fractional crystallisation of the desired (*R,R*)-amine, e.g. by precipitation as an acid addition salt;

C: Regeneration of pure (*R,R*)-amine;

D: Condensation to an (*R,R*)-tertiary amine in the presence of an inert organic solvent and of a non-basic acid acceptor, preferably at elevated temperature (since the secondary amine (IV) is sterically hindered);

E: Reduction, preferably under mild conditions, e.g. with a borohydride in an organic solvent;

F: Removal of the O- and N-protecting groups, preferably simultaneously by hydrogenolysis;

G: Resolution of the diastereoisomeric mixture (VIII) and isolation of the (*R,R*)-stereoisomer as free base or as an acid addition salt.

The invention therefore provides a process for the preparation of (−)-5-(*R*)-1-hydroxy-1-[(*R*)-1-methyl-phenylpropyl]aminoethyl salicylamide or an acid addition salt thereof, including the step of reduction of the glycyl carbonyl function of a 2-(hydroxy or protected hydroxy)-5-{N-protected-N-[(*R*)-1-methyl-3-phenylpropyl]glycyl}benzamide; characterised in that the N-protecting group has the formula

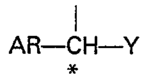

*(R)*

wherein Ar and Y are as hereinbefore defined.

This process preferably includes the sequence of steps:

(i) reduction of the glycl carbonyl function of a 2-(hydroxy or protected hydroxy)-5-{N-protected-N-[(*R*)-1-methyl-3-phenylpropyl]glycyl benzaminde;

(ii) deprotection of the 2-(hydroxy or protected hydroxy)-5-{(*R,S*)-1-hydroxy-2-[N-protected-N-[(*R*)-1-methyl-3-phenylpropyl]amino]ethyl}benzaminde; and

(iii) isolation of (−)-5-{(*R*)-1-hydroxy-2-[(*R*)-1-methyl-3-phenylpropyl]aminoethyl}salicylamide or an acid addition salt thereof.

The deprotection in step (ii) is preferably effected by hydrogenolysis, and the isolation in step (iii) preferably includes resolution with (−)-dibenzoyl-*d*-tartaric acid as resolving agent.

In this process, the 2-(protected hydroxy)-5-{N-protected-N-[(*R*)-1-methyl-3-phenylpropyl]glycyl}-benzamide is conveniently prepared by reaction of an N-[(*R*)-protected]-(*R*)-1-methyl-3-phenylpropylamine with a 4-0-protected-α-bromo-3-carbamoylacetophenone in the presence of a non-basic acid acceptor, preferably propylene oxide.

The N-[(*R*)-protected]-(*R*)-1-methyl-3-phenylpropylamine can be stereoselectively prepared by a) reaction of D-(+)-α-methylbenzylamine with benzyl acetone with concomitant or subsequent reduction, followed by b) resolution of the diastereomeric mixture by selective precipitation, conveniently by formation and selective precipitation of the (*R,R*) amine hydrochloride salt, and c) generation of the free (*R,R*) amine.

A preferred embodiment of the process according to the invention comprises the sequence:

1) reaction of an N-[(*R*)-protected]-(*R*)-1-methyl-3-phenylpropylamine with a 4-0-protected-α-bromo-3-carbamoylacetophenone in the presence of a non-basic acid acceptor to produce a 2-(0-protected)-5-{N-[(*R*)-protected]-N-[(*R*)-1-methyl-3-phenylpropyl]glycyl}benzamide;

2) reduction of the glycyl carbonyl function in the product from Step (1) to hydroxy, to yield a first diastereoisomeric mixture;

3) deprotection of the O and N functions of the product from Step (2), to yield a second disastereoisomeric mixture;

4) resolution of the second disastereoisomeric mixture from Step (3) by reaction with an acidic resolving agent to selectively precipitate the (*R,R*) stereoisomer as an acid addition salt thereof substantially free of the corresponding (*S,R*) stereoisomer; and, if desired, converting said acid addition salt of the (*R,R*)-stereoisomer into a different acid addition salt or into the free base; wherein the N-protecting group is as hereinbefore defined.

A particularly preferred embodiment of this process comprises:

1. reaction of N-[(*R*)-α-methylbenzyl]-(*R*)-1-methyl-3-phenylpropylamine with 4-benzyloxy-α-bromo-3-carbamoyl-acetophenone in the presence of propylene oxide to produce 2-benzyloxy-5-{(*R*)-α-methylbenzyl-N-[(*R*)-1-methyl-3-phenylpropyl]glycyl}benzamide;

2) reduction of the glycyl carbonyl function in the product from Step (1) to hydroxy by means of sodium borohydride;

3) removal of the protecting O and N functions of the product from Step (2) by hydrogenolysis; and

4) resolution of the deprotected product from Step (3) by reaction with (−)-dibenzoyl-*d*-tartaric acid to selectively precipitate the (*R,R*) stereoisomer as its dibenzoyl-*d*-tartaric acid salt substantially free of the corresponding (*S,R*) stereoisomer;

followed, if desired, by conversion of the dibenzoyl-*d*-tartrate acid salt into the hydrochloride salt or into the free base.

The compound N-[(*R*)-α-methylbenzyl]-(*R*)-1-methyl-3-phenylpropylamine is stereoselectively prepared by a) reductive condensation of D-(+)-α-methylbenzylamine with benzyl acetone followed by b) resolution of the diastereomeric mixture by selective precipitation of the (*R,R*) amine hydrochloride and c) generation of the free (*R,R*) amine

Compounds of the formula

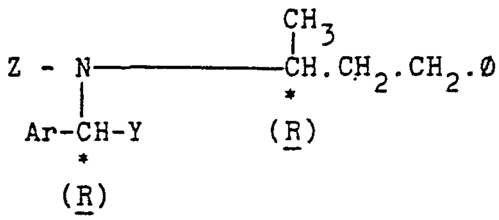

(XI)

wherein Ar and Y are as hereinbefore defined, and Z is a hydrogen atom or a group of the formula

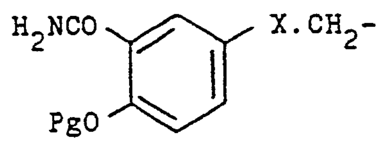

wherein Pg is an O-protecting group as hereinbefore defined and X is a carbonyl group or an (*R,S*) hydroxymethylene group, are novel intermediates and are features of this invention. Preferred compounds falling under formula (XI) are those in the foregoing reaction sequence, i.e. in which Ar is a phenyl group and Y is a methyl group, in particular

2-benzyloxy-5-{N-[(*R*)-α-methylbenzyl]-N-[(*R*)-1-methyl-3-phenylpropyl]glycyl}benzamide (VI in the reaction sequence);

2-benzyloxy-5-{(*R,S*)-1-hydroxy-2-[[(*R*)-1-methyl-3-phenylpropyl]-(*R*)-α-methylbenzylamino]ethyl}-benzamide (VII in the reaction sequence); and

N-[(*R*)-α-methylbenzyl]-(*R*)-1-methyl-3-phenylpropylamine (IV in the reaction sequence).

The steps A to G of the foregoing reaction scheme will now be described in detail with reference to preferred features:

Step A

D-(+)-α-Methylbenzylamine is reacted with benzylacetone, and the resulting Schiff's base is reduced to form amines (III). Conveniently the reaction is carried out under reducing conditions, e.g. by hydrogenating in the presence of Raney nickel, so that isolation of a Schiff's base intermediate is unnecessary. Usually the reaction will be carried out in an inert organic solvent, for example isopropanol. In an embodiment described in the Example below, reduction provides a diastereomeric mixture (*R,R and R,S*) containing about 75% by weight of the desired N-[(*R*)-α-methylbenzyl)-(*R*)-1-methyl-3-phenyl-propylamine (i.e. the *R,R* stereoisomer).

Step A1

N-[(*R*)-α-Methylbenzyl]-(*R,S*)-1-methyl-3-phenylpropylamine may also be prepared by first forming a Schiff's base in a first operation; and thereafter reducing the Schiff's base in a second distinct operation. The same starting materials can be used as in Step A. The Schiff's base can be formed by condensing D-(+)-α-methylbenzylamine with benzylacetone in a solvent mixture of toluene or xylene and toluene sulfonic acid with azeotropic removal of the water formed in the reaction. After removal of the solvent the crude imine is dissolved in a suitable solvent e.g. glacial acetic acid and reduced using Raney Nickel. The catalyst is then filtered off, the solvent is distilled off and the residue is distilled to yield the desired product as an (*R,R; R,S*) mixture.

Step B

The diastereomeric mixture from Step A or A1 can be readily resolved by formation of an acid addition salt by conventional methods. For instance the hydrogen chloride salt of the *R,R* stereoisomer can be selectively precipitated from a solution of the diastereomeric mixture in an organic solvent by bubbling hydrogen chloride therethrough or preferably by adding thereto a solution of hydrogen chloride (gas) in isopropanol.

Step C

The *R,R* amine hydrochloride resulting from Step B can be converted into the free base by conventional methods for instance by reaction with aqueous sodium hydroxide in e.g. an organic solvent for instance toulene, usually under an inert atmosphere e.g. nitrogen, followed by working up in a conventional manner.

Step D

The *R,R* amine free base from Step C is reacted with a 4-O-protected-α-bromo-3-carbamoylacetophenone to produce a 2-(O-protected)-5-{N-(*R*)-α-methylbenzyl]-N-[(*R*)-1-methyl-3-phenylpropyl]glycyl}-benzamide (VI). The reaction is caried out in the presence of a non-basic acid acceptor which is preferably propylene oxide. The reaction is conveniently carried out at elevated temperature, e.g. under reflux conditions in an inert organic solvent, for exmaple dimethylformamide. It is desirable, for best results, to conduct the reaction and subsequent work-up in the absence of light. The protecting group Pg is suitably any readily removable conventional hydroxy protecting group and is preferably benzyl.

Step E

Compound (VI) (from Step D) is reduced at the keto group, ideally under mild conditions which do not affect the protecting group Pg or the α-methylbenzyl group attached to and protecting the nitrogen atom, to produce a diastereomeric mixture of 2-benzyloxy-5-{(*R,S*)-1-hydroxy-2-[[(*R*)-1-methyl-3-phenylpropyl]-(*R*)-α-methyl-benzylamino]ethyl}benzamide (VII). Generally speaking, suitable reducing conditions will be well known to persons skilled in the art. One convenient procedure is to reduce with sodium borohydride or sodium cyanoborohydride, the reaction being conducted in an organic solvent, especially an alkanol e.g. ethanol or methanol. Lithium borohydride or an alkali metal alkylborohydride, e.g. lithium or potassium tri-*s*-butylborohydride or lithium or potassium tri-(3-methyl-2-butyl)borohydride, in an organic solvent such as tetrahydrofuran, can also be used.

Step F

The product (VII) of Step E is an isomeric mixture (*R,R,R* and *S,R,R*) containing about 90% by weight of the desired *R,R,R* isomer. It is treated to effect removal of the hydroxy protecting group Pg and N-protecting α-methylbenzyl group to afford the product (VIII) as a diastereomeric mixture, 5-{(*R,S*)-1-hydroxy-2-[(*R*)-1-methyl-3-phenylpropylamino]ethyl}salicylamide. Suitable conditions will be known to the skilled man in the art. We prefer to hydrogenate in the presence of a palladium-on-carbon catalyst.

Step G

*G1.* Resolution of the deprotected product (VIII) from Step F is usually carried out by treating it in an organic solvent, e.g. ethanol, with a suitable resolving agent such as dibenzoyl-*d*-tartaric acid monohydrate and allowing crystals to form, usually at room temperature. The first crop of crystals is the desired (*R,R*)-isomer of labetalol in the form of its salt, e.g. the dibenzoyl-*d*-tartrate acid salt (which is indeed an acid salt, containing equimolar amounts of the (*R,R*)-isomer and of the dibenzoyl-*d*-tartaric acid). It is usually desirable to seed the starting solution with a previously prepared *R,R* stereoisomer dibenzoyl-*d*-tartaric acid salt.

*G2.* The hydrochloride salt can be formed by the conventional technique of adding a solution of hydrogen chloride gas in an organic solvent e.g. ethanol to a solution or slurry of the acid addition salt obtained from G1. After allowing the reaction to proceed for a suitable period, the resulting solids can be filtered off and worked up in a conventional manner to yield the desired (*R,R*) isomer of labetalol as its hydrochloride salt.

If desired, the acid addition salt i.e., either the dibenzoyl-*d*-tartrate or hydrogen chloride salt, can be further treated by conventional techniques to provide the free (*R,R*)-amine base or some other acid addition salt.

Example

*A.* Into a Paar hydrogenation bottle charge: 121.2 g. (1.00 mole) of D-(+)-α-methylbenzylamine, 173.4 g. (1.17 mole, 17% excess) of benzylacetone, 60.1 g. (1.00 mole) of glacial acetic acid and 600 ml. of isopropanol. Add 80 g. (wet weight) of Raney nickel (Grace No. 28), washed with water to neutrality and then with isopropanol to remove the water. Hydrogenate the mixture under 4 atmospheres' hydrogen pressure (60 psi).

Filter the resulting mixture through a short bed of "Celite"® (infusorial earth). Wash the catalyst and "Celite"® with 3 × 100 ml. of isopropanol. Combine the isopropanol solutions and dilute with a further 660 ml. of isopropanol.

*B.* To this solution add, with efficient stirring over a period of about 30 minutes, 467 ml. of a 2.25N (1.05 mole) solution of dry hydrogen chloride in isopropanol; after about half has been added, the hydrochloride starts precipitating. The temperature rises to 33°C. Cool to ambient temperature and stir for 3 hours. Filter off the solid and wash it on the filter with 100 ml. of isopropanol. Dry the resulting solid and wash it on the filter with 100 ml. of isopropanol. Dry the resulting solid at 50°C. to yield a white solid m.p. 216.5—218°C., $[\alpha]_D^{26} = +59.6°$ (1% in MeOH); about 70% yield.

Recrystallize the solid from isopropanol using 15 ml. of solvent per gram of material. Cool to ambient temperature and stir for 3 hours. Filter, and wash the solid on the filter with 2 × 100 ml. isopropanol. Dry the solid at 50°C. to constant weight to obtain a white cyrstalline solid, m.p. 219—220.5°C., $[\alpha]_D^{26} = +60.8°$ (1% in MeOH); 61.8—64.5% yield.

*C.* Charge 78.0 g. (0.269 mole) of the *R,R*-amine-hydrochloride salt, 390 ml. of toluene and 135 ml. of 3M aqueous sodium hydroxide (0.405 mole, 50% excess) into a vessel and heat at 80°C. with stirring under nitrogen for 1 hour. Separate the resulting layers. Extract the water layer with 80 ml. of toluene. Combine

the toluene extracts and wash with 2 × 80 ml. saturated aqueous sodium bicarbonate (pH of last washing should not be greater than 9), with 2 × 80 ml. of water (last washing should be neutral) and 80 ml. saturated brine. Dry the toluene extracts over anhydrous sodium sulfate, filter and remove the solvent *in vacuo.* Distil the product at 128—134°C./120 Pa (0.9 mm. Hg.). A clear colorless liquid, 67.3 g. (98.7% yield), $[\alpha]_D = +78.4°$ (neat) or $[\alpha]_D = +115.2°$ (10% in MeOH) is obtained.

*D.* In a 1 liter three-necked flask equipped with stirrer, thermometer and reflux condenser charge 200 ml. of dimethylformamide (dried over 4A molecular sieves), 83.6 g. (0.24 mole) of 2-benzylloxy-5-bromoacetyl-benzamide, 50.7 g. (0.2 mole) of the amine from Step C and 35 ml. (29 g.; 0.5 mole) of propylene oxide.

Heat the stirred suspension to 45—47°C. and maintain the resulting solution at this temperature for 24 hours. The color of the solution changes from yellow to orange and later to reddish orange. Protect the reaction mixture from light at all times during the reaction and the subsequent work-up.

After 24 hours remove a sample and check the completion of the reaction. When the reaction is complete, cool the reaction mixture to room temperature and pour it into a stirred mixture of 600 ml. of water and 200 ml. of methylene dichloride.

Separate the phases, extract the aqueous layer twice with 200 and 100 ml. of methylene dichloride and wash the combined organic layers with 3 × 200 ml. of water. Dry thoroughly over anhydrous sodium or magnesium sulfate, filter, wash the cake with 2 × 50 ml. of methylene dichloride and remove the solvent under reduced pressure (16 000 Pa (120 mm. Hg.) and water bath temperature of about 40°C.).

Dissolve the red, viscous residue in 500 ml. of 2B ethanol and proceed immediately to the next step. (2B ethanol, an industrially denatured ethanol, is 95% ethanol containing 0.5% (v/v) benzene).

*E.* Cool the solution of amino-ketone obtained in Step D to 5°C.±2°C. under a blanket of nitrogen in a 2 liter three-necked flask equipped with a mechanical stirrer and thermometer.

Add to the reaction mixture 7.6 g. (0.2 mole) of sodium borohydride in portions so as to maintain the temperature and to avoid violent frothing of evolving hydrogen. After the addition is complete, maintain the reaction mixture at 5°±2°C. for an hour, then let it slowly warm to room temperature and continue to stir it for 16—18 hours. Cool intermittently to avoid temperatures higher than 25°C.

Check for complete reduction by the absence of starting amino-ketone. After complete reaction, distil out about 350 ml. of ethanol under reduced pressure — approximately 16 000 Pa (120 mm. Hg.) — and up to a pot temperature of 40°C.

Add 500 ml. of water and reflux the mixture for 1 hour (pot temperature about 86°C.), and then distill off about 150 ml. of ethanol at atmospheric pressure. Cool the reaction mixture to 40°C. and extract the yellow, soft resin that separates with 300 ml. of methylene dichloride. Cool the two-phase mixture to room temperature and separate and extract the aqueous layer with 125 ml. of methylene dichloride. Wash the combined organic layers with 2 × 200 ml. of water, and dry them over anhydrous sodium or magnesium sulfate, filter and wash the cake with 2 × 50 ml. of methylene dichloride.

Remove the solvent under reduced pressure and dissolve the residue in 650 ml. of ethanol. Add 12 g. (11.5 ml., 0.2 mole) of acetic acid and 6.25 g. of "Darco"®G—60 (activated carbon), heat to 65°C. and stir for 10 minutes at 65°C. Filter hot through a "Celite" bed, and wash the resulting cake with 3 × 150 ml. of hot ethanol; the resulting ethanol solution is used in Step F.

*F.* Charge the ethanol solution obtained in Step E into a hydrogenation flask, cool the contents in an icebath and add, under a blanket of nitrogen, 10 g. of 5% Pd/C. Allow the solution to warm to room temperature and reduce in a Paar apparatus with hydrogen under about 4 atmospheres' pressure 4.1 bar (60 psi).

After the reduction is complete, remove the catalyst by filtering the solution through a "Celite"® bed and wash the cake with 2 × 125 ml. of ethanol.

*G1.* Add to the filtrate a solution of 75.28 g. (0.2 mole) of dibenzoyl-*d*-tartaric acid (DB-*d*-TA) monohydrate in 200 ml. of ethanol. Seed with good quality salt and stir the mixture at room temperature for 3 days. A fine white precipitate slowly forms.

Filter off the precipitated salt, wash it with 125 ml. of ice-cold ethanol and dry it in a draft oven at 50°C. A white powder is obtained; m.p. (different batches:) 168—170°C. to 173.5—175°C., uncorrected; 58—60% yield from beginning of Step D.

Recrystallize the crude salt from 1400 ml. of boiling 90% aqueous ethanol. After dissolution is complete, cool to room temperature, seed with the desired *R,R* DB-*d*-TA salt and stir the resulting suspension for 16—18 hours. Filter off the precipitate, wash with 2 × 100 ml. of 90% aqueous ethanol and dry in a draft oven at 50°C. 71.5—73 g. of purified salt is obtained (m.p. 175—176°C., uncorrected).

*G2.* Into a three-necked round bottom flask equipped with a mechanical stirrer, thermometer, addition funnel and nitrogen bubbler charge: 6.87 g. (0.01 mole) of pulverized DB-*d*-TA salt of the *R,R*-stereoisomer obtained from Step F and 103 ml. of isopropanol. Stir the mixture for about 30—45 minutes until a paste-like but very fluid and easily stirrable mixture is obtained. Add, in a fast streamn, 4.8 ml. of a 2.19N solution of hydrogen chloride gas in ethanol (0.0105 mole; 5% excess). Stir the mixture efficiently at ambient temperature for about 6 hours. Filter off the solid and wash it on a filter with 3 × 12.5 ml. of isopropanol. Dry it in a draft oven at 50°C. to constant weight to obtain the hydrochloride salt of the *R,R* stereoisomer as a colorless solid, 3.43—3.46 g., m.p. 192—193°C., $[\alpha]_D = -15.3°$; −15° (DMF), C = 1); diasteromeric purity: 98.9%, >99% (different batches).

**Claims**

1. A process for the preparation of (−)-5-{(*R*)-1-hydroxy-2-[(*R*)-1-methyl-3-phenyl-3-phenylpropyl]-aminoethyl}salicylamide or an acid addition salt thereof, including the step of reduction of the glycyl carbonyl function of a 2-(hydroxy or protected hydroxy)-5-{N-protected-N-[(*R*)-1-methyl-3-phenylpropyl]-glycyl}benzamide; characterized in that the N-protecting group has the formula

|
AR—CH—Y
*
(*R*)

wherein Ar is an aryl group, especially one with a single benzene ring, and Y is a lower alkyl group having 1 to 6, preferably 1 to 4, carbon atoms, in particular wherein the N-protecting group is the (*R*)-α-methylbenzyl group.

2. A process as claimed in claim 1 wherein there is present an O-protecting group that can be removed by hydrogenolysis, in particular a benzyl group.

3. A process as claimed in claim 1 or claim 2 wherein the reduction is effected by means of a borohydride in an organice solvent, especially by sodium borohydride in ethanol.

4. A process as claimed in any of claims 1 to 3 which includes the sequence of steps

(i) reduction of the glycyl carbonyl function of a 2-(hydroxy or protected hydroxy)-5-{N-protected-N-[(*R*)-1-methyl-3-phenylpropyl]glycyl}benzamide;

(ii) deprotection, especially by hydrogenolysis, of the 2-(hydroxy or protected hydroxy)-5-{(*R,S*)-1-hydroxy-2-[N-protected-N-[(*R*)-1-methyl-3-phenylpropyl]amino]ethyl}benzamide; and

(iii) isolation of (−)-5-{(*R*)-1-hydroxy-2-[(*R*)-1-methyl-3-phenylpropyl]aminoethyl}salicylamide or an acid addition salt thereof, especially by resolution with (-)-dibenzoyl-*d*-tartaric acid as resolving agent.

5. A process as claimed in any of claims 1 to 4 wherein the 2-(protected hydroxy)-5-{N-protected-N-[(*R*)-1-methyl-3-phenylpropyl]glycyl}benzamide is prepared by reaction of an N-[(*R*)-protected]-(*R*)-1-methyl-3-phenylpropylamine with a 4-O-protected-α-bromo-3-carbamoylacetophenone in the presence of a non-basic acid acceptor, especially propylene oxide.

6. A process as claimed in claim 5 wherein the N-[(*R*)-protected]-(*R*)-1-methyl-3-phenylpropylamine is stereoselectively prepared by a) rection of D-(+)-α-methylbenzylamine with benzyl acetone with concomitant or subsequent reduction, followed by b) resolution of the diastereomeric mixture by selective precipitation, in particular by formation and selective precipitation of the (*R,R*) amine hydrochloride salt, and c) generation of the free *(R,R)* amine.

7. A process as claimed in any of claims 1 to 6 which comprises the sequence:

1) reaction of an N-[(*R*)-protected]-(*R*)-1-methyl-3-phenylpropylamine with a 4-O-protected-α-bromo-3-carbamoylacetophenone in the presence of a non-basic acid acceptor to produce a 2-(O-protected)-5-{N-[(*R*)-protected]-N-[(*R*)-1-methyl-3-phenylpropyl)glycyl}benzamide;

2) reduction of the glycyl carbonyl function in the product from Step (1) to hydroxy, to yield a first diastereomeric mixture;

3) deprotection of the O and N functions of the product from Step (2), to yield a second diastereoisomeric mixture;

4) resolution of the second diastereoisomeric mixture from Step (3) by reaction with an acidic resolving agent to selectively precipitate the (*R,R*) stereoisomer as an acid addition salt thereof substantially free of the corresponding (*S,R*) stereoisomer; and, if desired, converting said acid addition salt of the (*R,R*)-stereoisomer into a different acid addition salt or into the free base; wherein the N-protecting group is as defined in claim 1.

8. A process as claimed in any of claims 1 to 6 which comprises the sequence:

1) reaction of N-[(*R*)-α-methylbenzyl]-(*R*)-1-methyl-3-phenylpropylamine with 4-benzyloxy-α-bromo-3-carbamoylacetophenone in the presence of propylene oxide to produce 2-benzyloxy-5-{(*R*)-α-methyl-benzyl-N-[(*R*)-1-methyl-3-phenylpropyl]glycyl}benzamide;

2) reduction of the glycyl carbonyl function in the product from Step (1) to hydroxy by means of sodium borohydride;

3) removal of the protecting O and N functions of the product from Step (2) by hydrogenolysis; and

4) resolution of the deprotected product from Step (3) by reaction with (-)-dibenzoyl-*d*-tartaric acid to selectively precipitate the (*R,R*) stereoisomer as its dibenzoyl-*d*-tartaric acid salt substantially free of the corresponding (*S,R*) stereoisomer; and, if desired, converting the dibenzoyl-*d*-tartarate acid salt into the hydrochlodride salt or into the free base.

9. A process as claimed in claim 8, wherein the compound N-[(*R*)-α-methylbenzyl]-(*R*)-1-methyl-3-phenylpropylamide is stereoselectively prepared by a) reductive condensation of D-(+)-α-methylbenzyl-amine with benzyl acetone followed by b) resolution of the diastereomeric mixture by selective precipitation of the (*R,R*) amine hydrochloride and c) generation of the free (*R,R*) amine.

10. Compounds of the formula

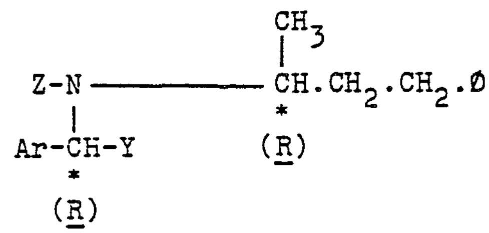

(XI)

wherein Ar is an aryl group and Y is a lower alkyl group having 1 to 6 carbon atoms, and Z is a hydrogen atom or a group of the formula

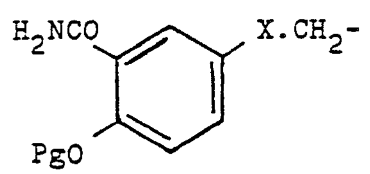

wherein Pg is a hydrogenolysable O-protecting group and X is a carbonyl group or an (*R,S*) hydroxymethylene group.

11. Compounds as claimed in claim 10, namely

2-Benzyloxy-5-{N-[(*R*)-α-methylbenzyl]-N-[(*R*)-1-methyl-3-phenylpropyl]glycyl}benzamide;

2-Benzyloxy-5-{(*R,S*)-1-hydroxy-2-[N-[(*R*)-1-methyl-3-phenylpropyl]-(*R*)-α-methylbenzylamino]ethyl}-benzamide; and

N-[(*R*)-α-Methylbenzyl]-(*R*)-1-methyl-3-phenylpropylamine.

**Patentansprüche**

1. Verfahren zur Herstellung von (−)-5-{(*R*)-1-Hydroxy-2-[(*R*)-1-methyl-3-phenylpropyl]aminoethyl}-salicylamid oder eines Säureadditionssalzes desselben, das den Schritt der Reduktion der Glycylcarbonyl-Funktion eines 2-(Hydroxy oder geschützten Hydroxy)-5-{N-geschützten-N-[(*R*)-1-methyl-3-phenylpropyl]-glycyl}-benzamids einschließt, dadurch gekennzeichnet, daß die N-Schutzgruppe die Formel

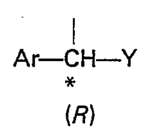

besitzt, in der

Ar eine Aryl-Gruppe, insbesondere eine mit einem einzelnen Benzol-Ring, ist und

Y eine Niederalkyl-Gruppe mit 1 bis 6, vorzugsweise 1 bis 4, Kohlenstoff-Atomen ist,

insbesondere in der die N-Schutzgruppe die (*R*)-α-Methylbenzyl-Gruppe ist.

2. Verfahren nach Anspruch 1, dadurch gekennzeichnet, daß eine O-Schutzgruppe anwesend ist, die durch Hydrogenolyse entfernt werden kann, insbesondere eine Benzyl-Gruppe.

3. Verfahren nach Anspruch 1 oder Anspruch 2, dadurch gekennzeichnet, daß die Reduktion mittels eines Borhydrids in einem organischen Lösungsmittel, insbesondere durch Natriumborhydrid in Ethanol durchgeführt wird.

4. Verfahren nach irgendeinem der Ansprüche 1 bis 3, das die Folge der Schritte.

(i) Reduktion der Glycylcarbonyl-Funktion eines 2-(Hydroxy oder geschützten Hydroxy)-5-{N-geschützten-N-[(*R*)-1-methyl-3-phenylpropyl]glycyl}benzamids,

(ii) Entfernung der Schutzgruppe, insbesondere durch Hydrogenolyse, des 2-(Hydroxy oder geschützten Hydroxy)-5-{N-geschützten-N-[(*R,S*)-1-hydroxy-2-[N-geschützten-N-[(*R*)-1-methyl-3-phenylpropyl]amino]ethyl}benzamids und

(iii) Isolierung von (−)-5-{(*R*)-1-Hydroxy-2-[(*R*)-1-methyl-3-phenylpropyl]aminoethyl}salicylamid oder eines Säureadditionssalzes desselben, insbesondere durch Auflösung mittels (−)-Dibenzoyl-d-weinsäure als Auflösungmittel, einschließt.

5. Verfahren nach irgendeinem der Ansprüche 1 bis 4, dadurch gekennzeichnet, daß das 2-(geschützte Hydroxy)-5-{N-geschützte-N-[(*R*)-1-methyl-3-phenylpropyl]-glycyl}benzamid durch Reaktion eines N-[(*R*)-geschützten]-(*R*)-1-methyl-3-phenylpropylamins mit einem 4-O-geschützten α-Bromo-3-carbamoyl-acetophenon in Gegenwart eines nicht-basischen Säureacceptors, insbesondere Propylenoxid, hergestellt wird.

6. Verfahren nach Anspruch 5, dadurch gekennzeichnet, daß das N-[(*R*)-geschützte]-(*R*)-1-methyl-3-phenylpropylamin stereoselektiv durch

a) Reaktion von D-(+)-α-Methylbenzylamin mit Benzylaceton mit gleichzeitiger oder nachfolgender Reduktion, gefolgt von

b) Auflösung des Diastereomeren-Gemischs durch selektive Fällung, insbesondere durch Bildung und selektive Fällung des (*R,R*)-Amin-hydrochlorid-Salzes, und

c) Erzeugung des freien (*R,R*)-Amins hergestellt wird.

7. Verfahren nach irgendeinem der Ansprüche 1 bis 6, das die Folge

1) Reaktion eines N-[(*R*)-geschützten]-(*R*)-1-methyl-3-phenylpropylamins mit einem 4-O-geschützten α-Bromo-3-carbamoylacetophenon in Gegenwart eines nicht-basischen Säureacceptors unter Bildung eines 2-(O-geschützten)-5-{N-[(*R*)-geschützten]-N-[(*R*)-1-methyl-3-phenylpropyl]glycyl}benzamids,

2) Reduktion der Glycylcarbonyl-Funktion zu Hydroxy in dem Produkt aus Schritt (1) unter Bildung eines ersten diastereomeren Gemischs,

3) Entfernung der Schutzgruppen von den O- und N-Funktionen des Produktes aus Schritt (2) unter Bildung eines zweiten diastereomeren Gemischs,

4) Auflösung des zweiten diastereomeren Gemischs aus Schritt (3) durch Reaktion mit einem sauren auflösenden Mittel zur selektiven Fällung des (*R,R*)-Stereoisomers als Säureadditionssalz desselben, das im wesentlichen frei von dem entsprechenden (*S,R*)-Stereoisomer ist, und gewünschtenfalls Überführen des Säureadditionssalzes des (*R,R*)-Stereoisomers in ein anderes Säureadditionssalz oder in die freie Base umfaßt.

8. Verfahren nach irgendeinem der Ansprüche 1 bis 6, das die Folge

1) Reaktion von N-[(*R*)-α-Methylbenzyl]-(*R*)-1-methyl-3-phenylpropylamin mit 4-Benzyloxy-α-bromo-3-carbamoylacetophenon in Gegenwart von Propylenoxid unter Bildung von 2-Benzyloxy-5-{(*R*)-α-methylbenzyl-N-[(*R*)-1-methyl-3-phenylpropyl]glycyl}benzamid,

2) Reduktion der Glycylcarbonyl-Funktion zu Hydroxy in dem Produkt aus Schritt (1) mit Hilfe von Natriumborhydrid,

3) Entfernung der Schutzgwuppen von den O- und N-Funktionen des Produkts aus Schritt (2) durch Hydrogenolyse,

4) Auflösung des von Schutzgruppen befreiten Produkts aus Schritt (3) durch Reaktion mit (−)-Dibenzoyl-*d*-weinsäure zur selektiven Fällung des (*R,R*)-Stereoisomers als Dibenzoyl-*d*-weinsäure-Salz desselben, das im wesentlichen frei von dem entsprechenden (*S,R*)-Stereoisomer ist, und gewünschtenfalls Überführen des Dibenzoyl-*d*-tartrat-Salzes in das Hydrochlorid-Salz oder in die freie Base umfaßt.

9. Verfahren nach Anspruch 8, dadurch gekennzeichnet, daß die Verbindung N-[(*R*)-α-Methylbenzyl]-(*R*)-1-methyl-3-phenylpropylamin stereoselektiv durch

a) reduktive Kondensation von D-(+)-α-Methylbenzylamin mit Benzylaceton, gefolgt von

b) Auflösung des Diastereomeren-Gemischs durch selektive Fällung des (*R,R*)-Amin-hydrochlorids und

c) Erzeugung des freien (*R,R*)-Amins hergestellt wird.

10. Verbindungen der Formel

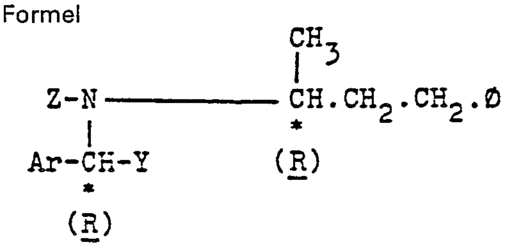

(XI)

in der

Ar eine Aryl-Gruppe ist und

Y eine Niederalkyl-Gruppe mit 1 bis 6 Kohlenstoff-Atomen is und

Z ein Wasserstoff-Atom oder eine Gruppe der Formel

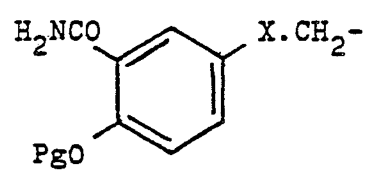

ist, worin

Pg eine hydrogenolysierbare O-Schutzgruppe ist und

X eine Carbonyl-Gruppe oder eine (*R,S*)-Hydroxymethylen-Gruppe ist.

11. Verbindungen nach Anspruch 10, nämlich

2-Benzyloxy-5-{N-[(*R*)-α-methylbenzyl]-N-[(*R*)-1-methyl-3-phenylpropyl]glycyl}benzamid,

2-Benzyloxy-5-{(*R,S*)-1-hydroxy-2-[N-[(*R*)-1-methyl-3-phenylpropyl]-(*R*)-α-methylbenzylamino]ethyl}-benzamid und

N-[(*R*)-α-Methlybenzyl]-(*R*)-1-methyl-3-phenylpropyl]amin.

**Revendications**

1. Procédé pour la préparation de (−)-5-{(*R*)-1-hydroxy-2-[(*R*)-1-méthyl-3-phénylpropyl]aminoéthyl}-saicylamide ou son sel d'addition d'acide, comprenant l'étape de réduction de la fonction glycyl carbonyle d'un 2-(hydroxy ou hydroxy protégé)-N-{N-protégé-N-[(*R*)-1-méthyl-3-phenylpropyl]glycyl}benzamide; caractérisé en ce que le groupe N-protecteur a pour formule

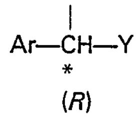

où Ar est un groupe aryle, en particulier ayant un seul noyau benzène, et Y est un groupe alcoyle inférieur ayant 1 à 6, de préférence 1 à 4 atomes de carbone, en particulier où le groupe N-protecteur est le groupe (*R*)-α-méthylbenzyle.

2. Procédé selon la revendication 1 où un groupe O-protecteur est présent qui peut être retiré par hydrogénolyse, en particulier un groupe benzyle.

3. Procédé selon la revendication 1 ou la revendication 2 où la réduction est effectuée au moyen d'un borohydrure dans un solvant organique en particulier par du borohydrure de sodium dans l'éthanol.

4. Procédé selon l'une quelconque des revendications 1 à 3 qui comprend la séquence d'étapes:

(i) réduction de la fonction glycyl carbonyle d'un 2-(hydroxy ou hydroxy protégé)-5-{N-protégée-N-[(*R*)-1-méthyl-3-phénylpropyl]glycyl}benzamide;

(ii) suppression de la protection, en particulier par hydrogénolyse, du 2-(hydroxy ou hydroxy protégé)-5-{(*R,S*)-1-hydroxy-2-[N-protégé-N-(R)-1-méthyl-3-phénylpropyl]amino]éthyl}benzamide; et

(iii) isolement du (−)-5-{(*R*)-1-hydroxy-2-[(*R*)-1-méthyl-3-phénylpropyl]aminoéthyl}salicylamide ou son sel d'addition d'acide, en particulier par résolution avec l'acide (−)-dibenzoyl-d-tartrique comme agent de résolution.

5. Procédé selon l'une quelconque des revendications 1 à 4 où le 2-(hydroxy protégé)-5-{N-protégé-N[(*R*)-1-méthyl-3-phénylpropyl]glycyl}benzamide est préparé par réaction d'une N-[(*R*)-protégée]-(*R*)-1-méthyl-3-phénylpropylamine avec une 4-O-protégée-α-bromo-3-carbamoylacétophénone en présence d'un accepteur d'acide non basique, en particulier de l'oxyde de propylène.

6. Procédé selon la revendication 5 où la N-[(*R*)-protégée]-(*R*)-1-méthyl-3-phénylpropylamine est stéréosélectivement préparée par a) réaction de D-(+)-α-méthylbenzylamine avec de la benzyl acétone avec réduction concomitante ou subséquente suivie de b) résolution du mélange diastéréomère par précipitation sélective, en particulier par formation et précipitation sélective du sel de chlorhydrate de (*R,R*)amine et c) production de la (*R,R*)amine libre.

7. Procédé selon l'une quelconque des revendications 1 à 6 qui comprend la séquence:

1) réaction d'une N-[(*R*)-protégée]-(*R*)-méthyl-3-phénylpropylamine avec une 4-O-protégée-α-bromo-3-carbamoylacétophénone en présence d'un accepteur d'acide non basique pour produire un 2-(O-protégé)-5-{N-[(*R*)-protégé]-N-[(*R*)-1-méthyl-3-phénylpropyl]glycyl}benzamide;

2) réduction de la fonction glycyl carbonyle danss le produit de l'étape (1) en hydroxy, pour donner un premier mélange de diastéréoisomères;

3) suppression de la protection des fonctions O et N du produit de l'étape (2) pour donner un second mélange de diastéréoisomères;

4) résolution du second mélange de diastéréoisomères de l'étape (3) par réaction avec un agent acide de résolution pour précipiter sélectivement le stéréoisomère (*R,R*) sous la forme de son sel d'addition d'acide sensiblement exempt du stéréoisomère (*S,R*) correspondant;

et, si on le souhaite, conversion dudit sel d'addition d'acide du stéréoisomère (*R,R*) en un sel d'addition d'acide différent ou en base libre;

où le groupe N-protecteur est tel que défini à la revendication 1.

8. Procédé selon l'une quelconque des revendications 1 à 6 qui comprend la séquence:

1) réaction de N-[(*R*)-α-méthylbenzyl]-(*R*)-1-méthyl-3-phénylpropylamine avec de la 4-benzyloxy-α-bromo-3-carbamoylacétophénone en présence d'oxyde de propylène pour produire du 2-benzyloxy-5-{(*R*)-α-méthylbenzyl-n-[(*R*)-1-méthyl-3-phénylpropyl]glycyl}benzamide;

2) réduction de la fonction glycyl carbonyle dans le produit de l'étape (1) en hydroxy au moyen de borohydrure de sodium;

3) enlèvement des fonctions O et N protectrices du produit de l'étape (2) par hydrogénolyse; et

4) résolution du produit sans protection de l'étape (3) par réaction avec de l'acide (−)-dibenzoyl-*d*-tartrique pour précipiter sélectivement le stéréoisomère (*R,R*) sous la forme de son sel d'acide dibenzoyl-*d*-tartrique sensiblement exempt du stéréoisomère (*S,R*) correspondant;

et si on le souhaite conversion du sel d'acide dibenzoyl-*d*-tartrate en sel de chlorhydrate ou en base libre.

9. Procédé selon la revendication 8 où le composé N-[(*R*)-α-méthylbenzyl]-(*R*)-1-méthyl-3-phényl-propylamine est stéréosélectivement préparé par a) condensation par réduction de D-(+)-α-méthylbenzyl-

amine avec de la benzyl acétone avec ensuite b) résolution du mélange diastéréomère par précipitation sélective du chlorhydrate de (*R,R*)amine et c) production de la (*R,R*)amine libre.

10. Composés de formule

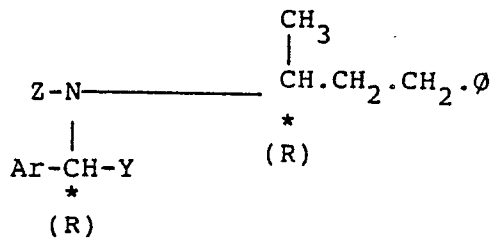

(XI)

où Ar est un groupe aryle et Y est un groupe alcoyle inférieur ayant 1 à 6 atomes de carbone et Z est un atome d'hydrogène ou un groupe de formule

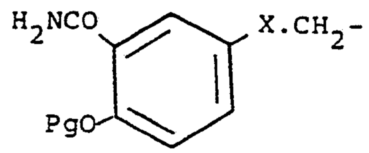

où Pg est un groupe O-protecteur hydrogénolysable et X est un groupe carbonyle ou un groupe (*R,S*)-hydroxyméthylène.

11. Composés selon la revendication 10, c'est-à-dire

2-Benzyloxy-5-{N-[(*R*)-α-méthylbenzyl]-N-[(*R*)-1-méthyl-3-phénylpropyl]glycyl}benzamide;

2-Benzyloxy-5-{(*R,S*)-1-hydroxy-2-[N-[-(*R*)-1-méthyl-3-phénylpropyl]-(*R*)-α-méthylbenzylamino]éthyl}-benzamide;

et N-[(*R*)-α-Méthylbenzyl]-(*R*)-1-méthyl-3-phénylpropylamine.